(19) **Europäisches Patentamt / European Patent Office / Office européen des brevets**

(11) **EP 1 488 775 B1**

(12) **EUROPEAN PATENT SPECIFICATION**

(45) Date of publication and mention
of the grant of the patent:
**25.04.2012 Bulletin 2012/17**

(21) Application number: **03715385.5**

(22) Date of filing: **20.03.2003**

(51) Int Cl.:
*A61K 8/31* (2006.01)   *A61K 8/37* (2006.01)
*A61K 8/39* (2006.01)   *A61K 8/86* (2006.01)
*A61Q 19/10* (2006.01)  *C11D 1/825* (2006.01)
*C11D 3/18* (2006.01)   *A61K 8/34* (2006.01)

(86) International application number:
**PCT/JP2003/003412**

(87) International publication number:
**WO 2003/080004 (02.10.2003 Gazette 2003/40)**

(54) **CLEANSING PREPARATION**

REINIGUNGSPRÄPARAT

PREPARATION DEMAQUILLANTE

(84) Designated Contracting States:
**DE FR GB**

(30) Priority: **26.03.2002 JP 2002085194**
**30.04.2002 JP 2002128364**
**12.08.2002 JP 2002234763**
**18.12.2002 JP 2002366767**
**18.12.2002 JP 2002366766**
**10.02.2003 JP 2003032078**

(43) Date of publication of application:
**22.12.2004 Bulletin 2004/52**

(73) Proprietor: **KAO CORPORATION**
**Tokyo 103-8210 (JP)**

(72) Inventors:
• **SHIMIZU, Masaki**
**Sumida-ku,**
**Tokyo 131-8501 (JP)**

• **TSUDA, Hiroko**
**Sumida-ku,**
**Tokyo 131-8501 (JP)**

(74) Representative: **HOFFMANN EITLE**
**Patent- und Rechtsanwälte**
**Arabellastraße 4**
**81925 München (DE)**

(56) References cited:
| | |
|---|---|
| EP-A- 0 758 641 | EP-A- 1 053 740 |
| WO-A-02/05758 | WO-A-95/03781 |
| JP-A- 3 161 428 | JP-A- 4 005 213 |
| JP-A- 4 224 507 | JP-A- 6 219 923 |
| JP-A- 8 040 827 | JP-A- 10 251 124 |
| JP-A- 11 035 421 | JP-A- 2000 136 114 |
| JP-A- 2002 241 224 | JP-A- 2003 012 456 |

Remarks:
The file contains technical information submitted after the application was filed and not included in this specification

## Description

### Field of the Invention

This invention relates to a cleansing preparation, and in more detail, to a cleansing preparation which is presented in a clear and beautiful liquid form, has excellent cleansing power, and causes no white turbidity even when applied to the skin wetted with water.

### Background of the Invention

As cleansing preparations that have conventionally been used to remove a makeup by washing away with water or by wiping off, there are various forms such as creams, emulsions, gels and solutions, all of which contain an oil ingredient added therein. These are generally produced by emulsifying or solubilizing the oil ingredient and water with a nonionic surfactant (for example, JP-A-3-161428, etc.).

However, these conventional oil-based cleansing preparations are not suited especially for the use in a bath room, because, when applied to the skin wetted with water, these preparations lose their original appearance due to emulsification or suspension caused by the presence of water. Also their cleansing power becomes weak against oily smears; and a feeling upon use could be deteriorated.

So far, numerous studies have been done in order to obtain a stable solubilized form, some of which were tried by adding polyols in a proportion of about 10 to 20 % or by use of a specific surfactant. However, no sufficiently acceptable product has been provided as yet, with regard to a cleansing preparation containing an oil as a principal ingredient, which is designed to be applied to the wet skin. In JP-A-4-5213 and JP-A-6-219923, for example, cleansing compositions which contain a specific nonionic surfactant, a water-soluble compound having a hydroxyl group, a liquid oil and water are proposed. The majority of these preparations have a viscosity of 300 mPa·s or higher, and are accompanied by the problem that they show poor spreadability and cleansing ability for oil-based makeup (especially, oil-based mascara). When applied to the wet skin in the bath room or the like, they form a rigid liquid crystalline structure by the admixture of water so that the cleansing power formakeup is significantly lowered as their viscosity increases and the cleansing power for oil-based makeup is also lowered as their white turbidity emerges. Accordingly, they can never be said to have sufficient water resistance, thus remaining to be fully unsatisfactory.

The present invention provides a water-resistant cleansing preparation presented in a clear liquid form, which develops neither a viscosity increase nor white turbidity upon contact with water, and which is neither lowered in cleansing power nor deteriorated in external appearance or feeling upon use.

### Summary of the Invention

The present inventors have found that the designing of an oil-based clear liquid preparation, which becomes neither turbid nor considerably viscous due to emulsification or suspension even when mixed with a significant amount of water, makes it possible to provide a cleansing preparation which is usable with wet hands and face in the bath room or at the washstand or in a similar setting. The cleansing composition is expected to develop neither a reduction in cleaning power nor a deterioration in external appearance or sensation upon application, and such a preparation can be achieved by combining specific surfactants with an oil.

The present invention provides a clear liquid cleansing preparation containing the following ingredients (A) to (E):

(A) at least one nonionic surfactant selected from:

(a) a monoglycerol fatty acid ester having a $C_{12-18}$ fatty acid residue,
(b) a polyglycerol fatty acid ester having a $C_{12-18}$ fatty acid residue,
(c) isostearyl pentaerythryl glyceryl ether, or
(d) a liquid nonionic surfactant having polyalkylene glycol chains and exhibiting IOB value of from 0.75 to 1.05,

(B) 5 to 30 wt.% of a nonionic surfactant other than the first-mentioned nonionic surfactant (A), and exhibiting IOB value of at least 1.1,
(C) 50 to 85 wt.% of a liquid oil ingredient,
(D) at most 12 wt.% of water, and
(E) 0.5 to 15 wt.% of a nonionic surfactant, higher alcohol; higher fatty acid or glycerol derivative, each having IOB value not greater than 0.6,
wherein the preparation is free of turbidity when 50 parts by weight of water is added to 100 parts by weight of the preparation, and wherein the IOB of the mixture of the ingredients (A), (B) and (E) is from 0.8 to 1.2.

**Detailed description of the Invention**

The ingredient (A) for use in the present invention is selected from the above-described compounds (a) to (d).

As the monoglycerol fatty acid ester (a) having a $C_{12\text{-}18}$ fatty acid residue, one in which the $C_{12\text{-}18}$ fatty acid residue has an unsaturated bond or a branched structure is preferred to inhibit its precipitation at low temperatures. Among them, glycerol monooleate, glycerol monolinoleate or glycerol monoisostearate is preferred.

As the polyglycerol fatty acid ester (b) having a $C_{12\text{-}18}$ fatty acid residue, an ester formed of a polyglycerol having a polymerization degree of from 2 to 6 glycerol units and a fatty acid is preferred, with a monoester formed by a polyglycerol having a polymerization degree of 2 and a fatty acid being more preferred from the standpoint of providing higher water resistance. Among them, diglyceryl monolaurate, diglyceryl monooleate or diglyceryl monoisostearate is preferred.

The liquid nonionic surfactant (d) having polyalkylene glycol chains and an IOB of from 0.75 to 1.05 is preferably one having an IOB of from 0.80 to 0.95 and containing polyalkylene glycol chains as hydrophilic groups. One having a liquid format roomtemperature is preferred because it hardly precipitates at any low temperature.

The term "IOB (inorganic/organic balance)" as used herein means an index which indicates a hydrophilicity-hydrophobicity balance, and in the present invention, is calculated using the following equation reported by Oda, Teramura, et al. Incidentally, an inorganic value and an organic value can be determined based on Organic Conceptional Diagram (FUJITA, Makoto: "Prediction of Organic Compounds and Organic Conceptional Diagram", Kagaku no Ryoiki (Fields of Chemistry), 11(10), 719-725 (1957)).

$$IOB = \frac{\text{Inorganic value}}{\text{Organic value}}$$

Examples of the nonionic surfactant (d) include polyoxyethylene (hereinafter referred to as "POE") fatty acid esters, POE alkyl ethers, fatty acid POE alkyl ethers, POE sorbitan fatty acid esters, POE sorbitol fatty acid esters, POE glycerol fatty acid esters, POE hydrogenated castor oils, and polyoxyethylene-polyoxypropylene alkyl ethers.

More specific examples include POE(6) oleyl ether (IOB = 0.83), POE (5) lauryl ether (IOB = 0.95), POE (15) lauryl ether stearate (IOB = 0.94), POE (8) dilaurate (IOB = 0.81), POE (12) diisostearate (IOB = 0.80), POE(12) dilaurate (IOB = 0.98), POE(30) sorbitol tetraoleate (IOB = 0.90), POE(6) monoisostearate (IOB = 0. 91), POE (6) monooleate (IOB = 0.90), POE(6) glyceryl monoisostearate (IOB = 0.84), POE(8) glyceryl monoisostearate(IOB=0.95),POE(30) glyceryltriisostearate (IOB = 0.98), and POE(30) glyceryl trioleate (IOB = 0.97).

As the ingredient (A), one or more compounds selected from the above-described compounds (a) to (d) can be used in combination. When the compounds (a) and (b) are used in combination, it is preferred to use them in such a way that weight ratio of (a) to (b) ranges of from 20:80 to 80:20, preferably from 25:75 to 50:50. Insofar as they are used in this range, no slimy touch lasts during rinsing, and moreover, a precipitate is hardly formed even at low temperatures.

As the ingredient (A), one or more of the compounds can be used. It is preferred to contain the ingredient (A) in a proportion of from 1 to 15 wt.% in the whole preparation. With respect to the compounds (a), (b) and (c), it is especially preferred to contain each of them in proportions of from 3 to 8 wt.%, for achievement of sufficient water resistance as well as good feeling upon use. As far as the compound (d) is concerned, it is preferred to contain it in a proportion of from 8 to 15 wt.% for achievement of sufficient water resistance.

The ingredient (B) for use in the present invention is a nonionic surfactant other than the ingredient (A), which is soluble in water and has an IOB of 1.1 or higher, preferably of from 1.2 to 2.0. In case IOB is lower than 1.1, it is not possible to provide either sufficient water resistance or refreshing wash-away property.

Specific examples include POE mono fatty acid esters, POE glycerol fatty acid esters (esters between glycerol, on which POE chains are added, and fatty acids), POE glyceryl mono fatty acid esters (POE chains are added on a glycerol mono fatty acid ester), alkyl glycosides, and POE sorbitan fatty acid esters. More preferred are POE mono fatty acid esters, POE glycerol fatty acid esters, POE glyceryl mono fatty acid esters and alkyl glycosides, as they can contribute to give a low viscosity and an excellent water resistance to the preparations. More specific examples include POE monolaurate (IOB = 1.34), POE glycerol mono coconut oil fatty acid (IOB = 1.22) and alkyl glucoside containing a $C_{9\text{-}11}$ alkyl chain and a saccharide having condensation degree of from 1 to 2 (IOB = 1.84), on account of their good cleansing power for colored cosmetics such as lipstick even in the presence of water (when performing massage on the wet skin and when washing them off) . The addition of these ingredients makes it possible to obtain an oil-based cleansing preparation having a high water resistance and excellent cleansing power for a wide variety of makeups.

As the ingredient (B), two or more nonionic surfactants can also be used. The ingredient (B) is contained in a proportion of from 5 to 30 wt.%, preferably from 7 to 16 wt.% in the whole preparation for achievement of sufficient water resistance and good wash-away property.

The liquid oil ingredient as the ingredient (C) shows fluidity at 25°C.

No particular limitation is imposed on such a liquid oil ingredient, insofar as it is commonly used in cosmetics. Usable examples include hydrocarbon oils such as liquid paraffin, liquid isoparaffin, polyisobutene, and squalane; ether oils such as hexadecyl-1,3-dimethyl butyl ether (see JP-A-9-87223); monoesters such as isopropyl palmitate, isopropyl myristate, cetyl 2-ethylhexanoate, and tridecyl isononanoate; triesters such as glyceryl tri-2-ethylhexanoate and glyceryl tri-caprylate/caprate; vegetable oils such as olive oil, jojoba oil, and macadamia nut oil; and silicone oils such as decamethyl cyclopentasiloxane and methylphenyl polysiloxane.

Of these, decamethyl cyclopentasiloxane and oils having from 20 to 30 carbon atoms and an inorganic value of 75 or smaller are preferred, as they are effective in solubilizing water. The term "inorganic value" as used herein means a value which is determined by Organic Conceptional Diagram employed upon determination of the above-described IOBs.

Examples of the oils, which have from 20 to 30 carbon atoms and an inorganic value of 75 or smaller, include liquid paraffin having 20 to 30 carbon atoms on average (e.g., "HICALL® K230", product of KANEDA Co., Ltd.; inorganic value = 0, average carbon number = 24), liquid isoparaffin (e.g., "PARMLEAM® EX", product of NOF Corporation; inorganic value = 0, average carbon number = 21), squalane (inorganic value = 0, average carbon number = 30), isopropyl palmitate (inorganic value = 60, carbon number = 19), isotridecyl isononanoate (inorganic value = 60, carbon number = 22), cetyl 2-ethylhexanoate (inorganic value = 60, carbon number = 24) and hexadecyl-1,3-dimethyl butyl ether (inorganic value = 20, carbon number = 22).

As the ingredient (C), two or more liquid oil ingredients can be used. The ingredient (C) is contained in a proportion of from 50 to 85 wt.%, preferably from 70 to 85 wt.% in the whole preparation for achievement of excellent cleansing power against oil-based makeups. Among such liquid oil ingredients, it is more preferred to contain decamethyl cyclopentasiloxane and/or an oil having 20 to 30 carbon atoms and an inorganic value of 75 or smaller in a proportion of from 40 to 85 wt.% in the whole preparation.

Water as the ingredient (D) is contained in a proportion of from 0 to 12 wt.%, preferably from 2 to 7 wt.% in the whole preparation. Containing water at a proportion greater than 12 wt.% may result in reduced clarity or phase separation with time, and therefore, is not preferred.

In the cleansing preparation according to the present invention, a nonionic surfactant, higher alcohol, higher fatty acid or glycerol derivative, all of which has an IOB value of 0.6 or lower, is additionally included as an ingredient (E). These compounds have an IOB of from 0.6 or lower, preferably from 0.25 to 0.6, more preferably from 0.3 to 0.6. The inclusion of the ingredient (E) can retain even higher water resistance, and can also reduce sliminess on skin upon rinsing.

Examples of such nonionic surfactants include diglyceryl dialkylates, propylene glycol mono fatty acid esters, and POE fatty acid esters. Specific examples include diglyceryl diisostearate (IOB = 0.41), diglyceryl dioleate (IOB = 0.42), propylene glycol monooleate (IOB = 0.39), propylene glycol monoisostearate (IOB = 0.38), propylene glycol monolaurate (IOB = 0.53), POE(6) diisostearate (IOB = 0.53), and POE(6) dioleate (IOB = 0.52).

Examples of the higher alcohol include those having 18 or less carbon atoms, such as myristyl alcohol (IOB = 0.36), isostearyl alcohol (IOB = 0.29) and oleyl alcohol (IOB = 0.28).

An example of the higher fatty acid is isostearic acid (IOB = 0.56).

An example of the glycerol derivative is isostearyl monoglyceryl ether (IOB = 0.53).

As the ingredient (E), two or more compounds can also be used. The ingredient (E) may be contained preferably in a proportion of from 0.5 to 15 wt.% in the whole amount of the preparation, within which from 2 to 8 wt. % is more preferred for achievement of excellent feeling upon rinsing.

In addition to the above-described ingredients, the cleansing preparation according to the present invention can also contain a polyhydric alcohol such as 1, 3-butylene glycol, propylene glycol or glycerol, a glycol ether such as ethyl carbitol or an alcohol such as ethanol to adjust the solubility of the nonionic surfactants and to improve the sensation upon application. It is preferred to control the content of such an alcohol ingredient below 5 wt.%, more preferably below 2 wt. % to obtain stable clear preparation, which does not undergo a phase separation with time.

It is also possible to include a viscosity increasing agent to adjust the viscosity, such as ultrafine silica particles, dextrin palmitate or organic bentonite; an organic acid or inorganic acid such as citric acid or phosphoric acid; and/or an inorganic salt, an anionic surfactant, a cationic surfactant, an amphoteric surfactant, a high molecular compound, an antimicrobial agent, an ultraviolet light absorber, an antioxidant, a chelating agent, a fragrance, a colorant, an extract, a medicament and/or the like.

The cleansing preparation according to the present invention can be obtained by appropriately mixing the predetermined ingredients together. Concerning raw materials which are solid at room temperature, the production can be facilitated by dissolving them beforehand and then combining all the ingredients into a homogeneous mixture.

The cleansing preparation according to the present invention is in a clear liquid form. The term "clear" as used herein indicates a state that a transmittance measured at a wavelength of 530 nm by a turbidity meter while using purified water as a reference is higher than the transmittance of a 0.001 wt.% aqueous suspension of titanium oxide. When a cleansing preparation contains a colorant, powder or the like added therein, the cleansing preparation shall be included within the scope of the present invention insofar as it is clear when measured without the colorant, powder or the like.

The term "liquid" as used herein indicates a state that a viscosity at 25°C is 1,000 mPa·s or lower (Brookfield rotational viscometer, Rotor No. 2, 30 rpm). At 25°C, the viscosity of the preparation is preferably 300 mPa·s or lower, more preferably 100 mPa·s or lower. Further, the viscosity at 25°C remains preferably 2,000 mPa·s or lower, more preferably 1,000 mPa·s or lower when water is added to and mixed with the preparation up to 50 parts by weight per 100 parts by weight of the preparation.

The cleansing preparation according to the present invention does not get turbid when 50 parts by weight of water is added to 100 parts by weight of the preparation. The term "turbid" as used herein means a state caused by emulsification or suspension. Transmittance of a cleansing preparation as measured at from 28 to 30°C and a wavelength of 530 nm by a turbidity meter (digital colorimeter"miniphoto® 5"; equipped with a glass cell of 10 mm in diameter; manufactured by Sanshin Kogyo Co., Ltd.) while using purified water as a reference is compared with the transmittance of a 0.02 wt.% aqueous suspension of titanium oxide ("Titanium Oxide JA-C", particle size: 0.1 to 0.5 μm or so; product of Tayca Corporation). When the transmittance of the cleansing preparation is lower than that of a 0.02 wt.% aqueous suspension of titanium oxide, the cleansing preparation is determined to be turbid.

Concerning the cleaning preparation according to the present invention, it is preferred that a temperature at which the preparation presents a clear or semi-clear form when up to 50 parts by weight of water is added to and mixed with 100 parts by weight of the preparation exists within a range of from 25 to 35°C. It is more preferred that the temperature at which the preparation takes a clear or semi-clear form when up to 100 parts by weight of water is added to and mixed with 100 parts by weight of the preparation lie within a range of from 28 to 32°C. It is even more preferred that the preparation take a clear or semi-clear form over the entire temperature range of from 28 to 32°C when up to 100 parts by weight of water is added to and mixed with 100 parts by weight of the preparation.

Expressing in other words, the term "clear or semi-clear form" as used herein means a state in which no white turbidity is present in the sense described above.

The following paragraph describes substantial reasons why the preparation is required tobe unemulsified and to remain in a clear or semi-clear, solubilized form until water is mixed up to a proportion of from 50 parts by weight to 100 parts by weight.

In the case of application of a facial cleansing oil, for example, about 1 g of water mixes in 2 g of the oil when the hands are lightly wet, and about 2 g of water mixes in 2 g of the oil when the hands are water-soaked. In the case of application of a body cleansing oil assuming that 3 g of the oil is applied to each arm, from about 1 to 1.5 g of water mixes in when the arm is lightly wet, and from about 2.5 to 3 g of water mixes in when the arm is water-soaked.

As the surface temperature of the skin is about 30°C (as measured by a surface thermometer), it is important for the preparation of the present invention to have a temperature range, in which its oil solubilizes water, around the skin surface temperature under the conditions that water mixes in under the above-described use environment, within from 25 to 35°C, more preferably from 28 to 32°C.

If the temperature range in which the oil solubilizes water does not exist within from 25 to 35°C and the clear or semi-clear form cannot be maintained, the use of the preparation on the wet skin develops turbidity, leading to deteriorations in massage feeling and external appearance. Further, the ability to dissolve oil impurities is reduced when the preparation turns to an O/W emulsion, and the rinsability is deteriorated when the preparation turns to a W/O emulsion.

In the present invention, it is important to control the IOB of the mixture of the ingredients (A), (B) and (E). The Mixed IOB is in a range of from 0.8 to 1.2, with a range of from 0.9 to 1.1 being preferred. If the mixed IOB is lower than 0.8, the preparation turns to a W/O emulsion when it is used on the wet skin and is mixed with water. As a consequence, the massage feeling and external appearance are deteriorated, and moreover, the rinsability is deteriorated. Therefore, a mixed IOB lower than 0.8 is not preferred. If the mixed IOB exceeds 1.2, the preparation turns to an O/W emulsion and develops turbidity when it is used on the wet skin and is mixed with water. As a consequence, the massage feeling and external appearance are deteriorated, and moreover, the ability to dissolve oil dirts and the massage feeling specific to oil are lost. Therefore, amixedIOBhigher than 1.2 is not preferred

The mixed IOB substantially takes a constant value provided that the total amount and kinds of the ingredients (A), (B) and (E) and the kind of the oil ingredient are determined. An appropriate mixed IOB can be determined by adjusting the proportions of the ingredients (A), (B) and (E) such that the overall system does not get turbid at about 30°C when 50 parts by weight of water is added to and mixed with 100 parts by weight of the preparation, and then by conducting a calculation based on the specific IOBs and proportions of the individual ingredients at the thus-selected mixing ratio. In the case of a surfactant a, surfactant b and surfactant c, for example, its mixed IOB canbe determined in accordance with the following equation:

$$\text{Mixed IOB} = \frac{\{IOB(a) \times wt.\% \text{ of } a + IOB(b) \times wt.\% \text{ of } b + IOB(c) \times wt.\% \text{ of } c\}}{\{wt.\% \text{ of } a + wt.\% \text{ of } b + wt.\% \text{ of } c\}}$$

When the ingredient (d) is used as the ingredient (A), the fraction of the ingredient (d) based on the total amount of the ingredients (d) and (B), that is, the weight ratio, the ingredient (d)/{the ingredient (d) + the ingredient (B)}, is preferably from 0.25 to 0.65, with from 0.35 to 0.45 being preferred. If this ratio becomes greater than the above-described range, the proportion of the surfactant ingredient (d), the rinsability of which is not very good, becomes greater compared with that of the water-soluble surfactant ingredient (B) which acts to improve the rinsability, so that a slimy feeling caused by the ingredient (d) tends to become stronger. If the ratio becomes smaller than the above-described range, it becomes difficult to provide the resulting preparation with sufficient water resistance.

In the present invention, it is preferred that the viscosity of the preparation remains below 300 mPa·s when from 50 parts by weight to 100 parts by weight of water is added to 100 parts by weight of the preparation. A viscosity higher than the above-described upper limit leads to a substantial difference in massage feeling between the use on the wet skin and the use on the dry skin, so that the preparation can hardly be used with comfort.

The cleansing preparation according to the present invention can be applied as a makeup remover by adding water thereto. As a method for adding water upon application, water can be added to the cleansing preparation before its application; the cleansing preparation can be dispensed onto the hand which has been moistened with water in advance; or the cleansing preparation can be applied to the wet face or hand in the bath room. The amount of water to be added may preferably range from 20 to 100 parts by weight based on 100 parts by weight of the cleansing preparation.

The cleansing preparation according to the present invention does not lose its cleansing power even when mixed with water, but on the contrary, its mixing with water provides an adequate massage feeling and reduces stickiness on the skin during massaging.

Examples

Examples 1-29 & Comparative Examples 1-18

Cleansing preparations of the formulations presented in Table 1 to Table 6 were produced, and the resultant cleansing preparations were ranked in external appearance, form, makeup removal, transmittance and makeup removal when mixed with water, rinsing property, and overall performance. The results are presented in Table 1 to Table 6.

(Production procedure)

Each cleansing preparation was produced by completely dissolving with heating the ingredients (A) and (E) and other ingredients in the ingredient (C), adding the ingredients (B) and (D), and then stirring them into a uniform mixture.

(Ranking methods)

(1) External appearance

A transmittance of each cleansing preparation was measured at 25°C and/or 40°C and a wavelength of 530 nm by a turbidity meter (digital colorimeter "mini photo® 5"; equipped with a glass cell of 10 mm in diameter; manufactured by Sanshin Kogyo Co., Ltd.) while using purified water as a reference. The transmittance was determined to indicate "clear" when it was higher than the transmittance of a 0.001 wt.% aqueous suspension of titanium oxide, while the transmittance was determined to indicate "semi-clear" when it was lower than the transmittance of the 0.001 wt.% aqueous suspension of titanium oxide but was higher than the transmittance of a 0.02 wt.% aqueous suspension of titanium oxide.

(2) Form

Each cleansing preparation was determined to be "liquid" when its viscosity as measured at 25°C by a Brookfield rotational viscometer (Rotor No. 2, 30 rpm) was 1,000 mPa·s or lower.

(3) Makeup removal

Removal of oil-basedmakeup: Amascara ("DialMascara®", product of MAYBELLINE LLC) was applied thinly over areas of 1.5 cm in diameter on the forearm, and was left for 3 hours to dryness. About 25 mg of the cleansing preparations (or their mixtures with 50 parts by weight of water) were placed on the areas, respectively, and each area was lightly massaged 30 times with a finger. The area was then rinsed with running water, and the result was visually observed as to how much of the mascara stays at each of the areas. The observation results were ranked in accordance with the below-described standards.

Removal of lipstick makeup: A lipstick ("AUBE Rouge Livelish® RS415", product of Kao Corporation) was applied thinly over areas of 1.5 cm in diameter on the forearm, and was left for 3 hours to dryness. About 25 mg of the cleansing preparations (or their mixtures with 50 parts by weight of water) were placed on the areas, respectively, and each area was lightly massaged 30 times with a finger. The area was then rinsed with running water, and the result was visually observed as to how much of the lipstick stays at each of the areas. The observation results were ranked in accordance with the following standards.

A: Makeup removal was good with quick lifting.
B: Makeup removal was good.
C: Makeup removal was fair.
D: Makeup removal was relatively poor.
E: Makeup removal was poor.

(4) Transmittance when mixed with water

With respect to each cleansing preparation, it was mixed with 10 parts by weight or 50 parts by weight of water to obtain a sample. Its transmittance was measured at a wavelength of 530 nm by a turbidity meter (digital colorimeter "mini photo® 5"; equipped with a glass cell of 10 mm in diameter; manufactured by Sanshin Kogyo Co., Ltd.) while using purified water as a reference. The transmittance was compared with the transmittances of aqueous suspensions of titanium oxide ("Titanium Oxide JA-C", particle size: 0.1 to 0.5 $\mu$m or so; product of Tayca Corporation), and ranked in accordance with the following standards.

A: Higher than the transmittance of the 0.001 wt.% aqueous suspension of titanium oxide.
B: Higher than the transmittance of the 0.008 wt.% aqueous suspension of titanium oxide, but lower than the transmittance of the 0.001 wt.% aqueous suspension of titanium oxide.
C: Higher than the transmittance of the 0.02 wt. % aqueous suspension of titanium oxide, but lower than the transmittance of the 0.008 wt.% aqueous suspension of titanium oxide.
D: Lower than the transmittance of the 0.02 wt.% aqueous suspension of titanium oxide.

In the present invention, it is preferred for each cleansing preparation to include, within a range of from 25 to 35°C that corresponds to an environment on the palm and face, a range in which the cleaning preparation does not get turbid even when water is mixed in as much as 50 wt.% of the own weight of the cleansing preparation. Each measurement was conducted shortly after adjusting the temperature of a mixture, which hadbeen obtained by combining 10 parts by weight or 50 parts by weight of purified water with 100 parts by weight of the relevant cleaning preparation, to from 28 to 30°C and then stirring the mixture into a uniform mixture.

(5) Rinsing property

The ease in rinsing each cleansing preparation was ranked when it was applied as much as about 2 g to the forearm and then washed away with tepid water.

A: Easily washed away, and no slimy feeling remained.
B: Easily washed away.
C: Hardly washed away.
D: Oil remained on the skin despite its removal was attempted for a long time, and unable to wash it away.

(6) Overall performance

Each cleaning preparation was used and ranked by ten expert panelists. Firstly, a lipstick and an oil-based mascara were applied to the face, and were then left for about 3 hours to dryness. About 2 g of the cleansing preparation was dispensed onto a hand from which water had been gently swished off after soaking it well with water once. The cleansing preparation was spread over the entire face while looking at herself in a mirror. After the cleansing preparation was interfused with the makeup, it was washed away with tepid water. Overall ranking of the appearance, makeup removal and rinsing property was conducted in accordance with the following standards.

A: Eight or more out of 10 answered "good".
B: Six to seven out of 10 answered "good".
C: Four to five out of 10 answered "good".
D: Three or fewer out of 10 answered "good".

Examples 7, 10, 13 and 29 do not form part of the invention.

Table 1

| Ingredient (wt.%) | | | Example | | | | | | | |
|---|---|---|---|---|---|---|---|---|---|---|
| | | | 1 | 2 | 3 | 4 | 5 | 6 | 7 | 8 |
| A | Glycerol monooleate ("EXCEL® O-95R, prod. of Kao Corp.) | IOB=0.62 | 3.70 | 3.70 | | | | | 6.00 | 5.97 |
| | Glycerol monoisostearate ("GWIS-100", prod. of Nihon Emulsion Co., Ltd.) | IOB=0.63 | | | | | 6.50 | | | |
| | Sufflower monoglyceride ("Sunsoft® No. 8090", prod. of Taiyo Kagaku Co., Ltd.) 1) | IOB=0.66 | | | | | | 4.63 | | |
| | Isostearyl pentaerythryl glyceryl ether ("LC-2", prod. of Kao Corp.) | IOB=0.92 | 1.05 | 1.05 | 3.50 | 3.50 | | | | |
| B | POE(7) glyceryl mono(coconut fatty acid) ("UNIGLY® MK-207, prod. of NOF Corp.) | IOB=1.22 | | | | | | 16.54 | | |
| | Polyoxyethylene monolaurate ("EMANON® 1112", prod. of Kao Corp.) | IOB=1.34 | 14.00 | 14.00 | 14.00 | 14.00 | 11.50 | | 14.20 | 14.10 |
| C | Decamethyl cyclopentasiloxane ("Silicone SH245", prod. of Dow Corning Toray Silicone Co., Ltd.) | | 21.00 | 20.00 | 25.00 | 23.00 | 21.50 | | 20.00 | 19.94 |
| | Isotridecyl isononanoate ("SALACOS® 913", prod. of The Nisshin Oil Mills Ltd.) | C=22, I.V.=60[2] | | | | | | 30.62 | | |
| | Liquid paraffin ("HICALL® K350", prod. of KANEDA Co., Ltd.) | C=35, I.V.=0 | 16.50 | 15.85 | 19.00 | 21.00 | 15.80 | | 12.00 | 13.04 |
| | Liquid paraffin ("HICALL® K230", prod. of KANEDA Co., Ltd.) | C=24, I.V.=0 | 36.82 | 31.02 | 23.00 | | 39.00 | 45.90 | 39.80 | 29.93 |
| | Isopropyl palmitate ("EXCEPARL® IPP", prod. of Kao Corp.) | C=19, I.V.=60 | | 5.80 | 8.00 | 30.00 | | | 5.00 | 14.93 |
| D | Purified water | | 3.60 | 3.50 | 1.00 | | 1.00 | 0.98 | 3.00 | 1.09 |
| E | Diglycerol diisostearate ("COSMOL® 42", prod. of The Nisshin Oil Mills Ltd.) | IOB=0.41 | | | | | 6.00 | | | |
| | Myristyl alcohol ("KALCOL® 4098", prod. of Kao Corp.) | IOB=0.36 | 1.90 | 1.90 | 3.50 | | 4.00 | 1.33 | | |
| | Isostearic acid (prod. of Nissan Chemical Industries, Ltd.) | IOB=0.56 | 0.68 | 0.68 | 0.50 | | 0.70 | | | 1.00 |
| Others | 2-Ethylhexyl glyceryl ether (89%) ("GE-EH", prod. of Kao Corp.) | IOB=1.05 | 0.75 | 0.75 | 2.50 | 2.50 | | | | |
| | POE(8) diisostearate ("EMALEX® 400di-IS", prod. of Nihon Emulsion Co., Ltd.) | IOB=0.63 | | | | | | | | |
| | Sorbitan monostearate ("RHEODOL® SP-S10", prod. of Kao Corp.) | IOB=0.93 | | | | | | | | |
| | POE(6) diisostearate ("EMALEX® 300di-IS", prod. of Nihon Emulsion Co., Ltd.) | IOB=0.53 | | | | | | | | |
| | Propylene glycol | | | 1.75 | | | | | | |

1) Contained 60 to 80 wt.% of Glycerol monolinoleate, 2) I.V.: Inorganic Value.

EP 1 488 775 B1

Table 1 (Cont'd)

| | Example | | | | | | | |
|---|---|---|---|---|---|---|---|---|
| | 1 | 2 | 3 | 4 | 5 | 6 | 7 | 8 |
| External appearance (25°C) | Clear | Clear | Clear | Clear | Clear | Clear | Clear | Clear |
| (40°C) | Clear | Clear | Clear | Clear | Clear | Clear | Clear | Clear |
| Form | Liq. | Liq. | Liq. | Liq. | Liq. | Liq. | Liq. | Liq. |
| Make removal (oil-based) | A | A | A | A | A | A | A | A |
| Transmittance (%) when mixed with water — Mixed with 10 wt. parts of water | A | A | A | A | A | A | C | A |
| Transmittance (%) when mixed with water — Mixed with 50 wt. parts of water | B | B | A | B | C | B | B | B |
| Makeup removal when mixed with 50 parts by weight of water (oil-based) | B | B | B | B | B | A | B | B |
| Makeup removal when mixed with 50 parts by weight of water (lipstick) | B | B | B | B | B | B | B | B |
| Rinsing property | A | A | A | B | A | B | B | B |
| Overall performance | A | A | A | B | A | B | B | B |

EP 1 488 775 B1

## Table 2

| Ingredient (wt.%) | | | Example | | | Comparative Example | | | | | |
|---|---|---|---|---|---|---|---|---|---|---|---|
| | | | 9 | 10 | 11 | 1 | 2 | 3 | 4 | 5 | 6 |
| A | Glycerol monooleate ("EXCEL® O-95R, prod. of Kao Corp.) | IOB=0.62 | 5.30 | | | 6.00 | | | | | |
| | Glycerol monoisostearate ("GWIS-100", prod. of Nihon Emulsion Co., Ltd.) | IOB=0.63 | | | | | | | | | |
| | Sufflower monoglyceride ("Sunsoft® No. 8090", prod. of Taiyo Kagaku Co., Ltd.) 1) | IOB=0.66 | | | | | | 6.34 | | | |
| | Isostearyl pentaerythryl glyceryl ether ("LC-2", prod. of Kao Corp.) | IOB=0.92 | | 8.05 | 7.90 | | | | | | |
| B | POE(7) glyceryl mono(coconut fatty acid) ("UNIGLY® MK-207, prod. of NOF Corp.) | IOB=1.22 | | | | | | | | | |
| | Polyoxyethylene monolaurate ("EMANON® 1112", prod. of Kao Corp.) | IOB=1.34 | 14.00 | 7.53 | 9.18 | | 20.25 | | 15.00 | 14.00 | 14.00 |
| C | Decamethyl cyclopentasiloxane ("Silicone SH245", prod. of Dow Corning Toray Silicone Co., Ltd.) | | 20.00 | 19.89 | 19.51 | 20.00 | 19.92 | 19.55 | 20.00 | 20.00 | 20.00 |
| | Isotridecyl isononanoate ("SALACOS® 913", prod. of The Nisshin Oil Mills Ltd.) | C=22, I.V.=60[2] | | | | | 10.04 | 21.47 | 16.00 | 15.00 | 15.00 |
| | Liquid paraffin ("HICALL® K350", prod. of KANEDA Co., Ltd.) | C=35, I.V.=0 | 15.00 | | | | | | | | |
| | Liquid paraffin ("HICALL® K230", prod. of KANEDA Co., Ltd.) | C=24, I.V.=0 | 33.75 | 39.66 | 38.03 | 44.00 | 29.86 | 24.63 | 26.00 | 28.00 | 33.40 |
| | Isopropyl palmitate ("EXCEPARL® IPP", prod. of Kao Corp.) | C=19, I.V.=60 | 8.00 | 24.87 | 24.38 | 30.00 | 8.03 | 22.90 | 11.00 | 13.80 | 10.00 |
| D | Purified water | | 2.00 | | | | 1.17 | 1.17 | | 2.20 | 1.50 |
| E | Diglycerol diisostearate ("COSMOL® 42", prod. of The Nisshin Oil Mills Ltd.) | IOB=0.41 | | | | | 9.94 | | | | |
| | Myristyl alcohol ("KALCOL® 4098", prod. of Kao Corp.) | IOB=0.36 | 1.20 | | 1.00 | | | 3.94 | 6.00 | | |
| | Isostearic acid (prod. of Nissan Chemical Industries, Ltd.) | IOB=0.56 | 0.75 | | | | 0.79 | | | 1.00 | |
| Others | 2-Ethylhexyl glyceryl ether (89%) ("GE-EH", prod. of Kao Corp.) | IOB=1.05 | | | | | | | | | |
| | POE(8) diisostearate ("EMALEX® 400di-IS", prod. of Nihon Emulsion Co., Ltd.) | IOB=0.63 | | | | | | | | 6.00 | |
| | Sorbitan monostearate ("RHEODOL® SP-S10", prod. of Kao Corp.) | IOB=0.93 | | | | | | | | 6.00 | |
| | POE(6) diisostearate ("EMALEX® 300di-IS", prod. of Nihon Emulsion Co., Ltd.) | IOB=0.53 | | | | | | | | | 6.10 |
| | Propylene glycol | | | | | | | | | | |

1) Contained 60 to 80 wt.% of Glycerol monolinoleate, 2) I.V.: Inorganic Value.

Table 2 (Cont'd)

| | Example | | | Comparative Example | | | | | |
|---|---|---|---|---|---|---|---|---|---|
| | 9 | 10 | 11 | 1 | 2 | 3 | 4 | 5 | 6 |
| External appearance (25°C) | Clear | Clear | Clear | Clear | Clear | Semi-clear | Semi-clear | Clear | Clear |
| (40°C) | Clear | Clear | Clear | Clear | Clear | Semi-clear | Semi-clear | Clear | Clear |
| Form | Liq. | Liq. | Liq. | Liq. | Liq. | Liq. | Liq. | Liq. | Liq. |
| Make removal (oil-based) | A | A | A | A | A | A | A | A | A |
| Transmittance (%) when mixed with water — Mixed with 10 wt. parts of water | A | B | A | D | A | D | B | D | C |
| Transmittance (%) when mixed with water — Mixed with 50 wt. parts of water | C | B | B | D | D | D | D | D | D |
| Makeup removal when mixed with 50 parts by weight of water (oil-based) | B | B | B | A | D | A | D | E | E |
| Makeup removal when mixed with 50 parts by weight of water (lipstick) | B | C | C | D | B | D | B | B | B |
| Rinsing property | A | B | B | D | B | D | B | B | B |
| Overall performance | A | B | B | D | D | D | D | D | D |

## Table 3

| Ingredient (wt.%) | | | Example | | | | | | | | |
|---|---|---|---|---|---|---|---|---|---|---|---|
| | | | 12 | 13 | 14 | 15 | 16 | 17 | 18 | 19 | 20 |
| A | Diglycerol monolaurate ("SY-GLYSTER®", prod. of Sakamoto Yakuhin Kogyo Co., Ltd.) | IOB=1.06 | 5.00 | | | | | | | | |
| | Diglycerol monooleate ("Sunsoft® Q17-B", prod. of Taiyo Kagaku) | IOB=0.80 | | | | 4.86 | | | | | |
| | Hexaglycerol monooleate ("Sunsoft® Q-17F", prod. of Taiyo Kagaku) | IOB=1.20 | | | | | 7.25 | | | | |
| | Diglycerol monoisostearate ("COSMOL® 41", prod. of The Nisshin Oil Mills) | IOB=0.79 | | 10.30 | 5.10 | | | 3.31 | 3.95 | 3.94 | 3.86 |
| | POE(3.3) mono(C$_{9-11}$ alkyl) ether ("SOFTANOL® 33", prod. of Nippon Shokubai Co., Ltd.) | IOB=0.77 | | | | | | 4.94 | 5.89 | 5.82 | 5.76 |
| | Isostearyl pentaerythryl glyceryl ether ("LC-2", prod. of Kao) | IOB=0.92 | | | | 0.97 | | 0.88 | 1.05 | 1.04 | 1.03 |
| B | Polyoxyethylene monolaurate ("EMANON® 1112HG", prod. of Kao) | IOB=1.34 | 11.00 | 12.81 | 13.12 | 14.11 | 6.09 | | | | 13.44 |
| | POE(7) glyceryl mono(coconut fatty acid) ("UNIGLY® MK-207, prod. of NOF) | IOB=1.22 | | | | | | 17.59 | | | |
| | POE(20) sorbitan monolaurate ("RHEODOL® Super TW-L120", prod. of Kao) | IOB=1.53 | | | | | | | 8.39 | 4.14 | |
| | Alkyl polyglucoside (C$_{9-11}$, saccharide condensation degree: 1.3, 40% aq. soln.; prod. of Kao) | IOB=1.84 | | | | | | | | 6.41 | |
| C | Decamethyl cyclopentasiloxane ("Silicone SH245", prod. of Dow Corning Toray Silicone) | | 21.50 | 19.71 | 20.19 | 20.91 | 18.55 | 16.60 | 19.79 | 19.72 | 19.34 |
| | Isopropyl palmitate ("EXCEPARL® IPP", prod. of Kao) | C=19, I.V.=60[1] | | | | | | 9.27 | | | |
| | Liquid paraffin ("HICALL® K350", prod. of KANEDA) | C=35, I.V.=0 | 15.80 | 15.77 | 16.15 | 15.37 | | 12.39 | 14.77 | 15.78 | 14.43 |
| | Liquid paraffin ("HICALL® K230", prod. of KANEDA) | C=24, I.V.=0 | 39.00 | 39.44 | 40.39 | 38.91 | 46.37 | 41.55 | 41.33 | 41.42 | 36.45 |
| D | Purified water | | 3.00 | 1.97 | 1.01 | 1.95 | 2.61 | 1.29 | 3.00 | | 3.50 |
| E | Isostearic acid (prod. of Nissan Chemical Industries) | IOB=0.56 | 0.70 | | | 0.97 | 2.61 | 0.84 | 1.00 | 0.99 | 0.98 |
| | Myristyl alcohol ("KALCOL® 4098", prod. of Kao) | IOB=0.36 | 4.00 | | 4.04 | 1.95 | | | | | |
| Others | Decaglycerol oleate ("Sunsoft® Q-17S", prod. of Taiyo Kagaku) | IOB=1.40 | | | | | | | | | |
| | Diglycerol diisostearate ("COSMOL® 42", prod. of The Nisshin Oil Mills) | IOB=0.41 | | | | | 7.25 | | | | |
| | 2-Ethylhexyl glyceryl ether (89% aq. soln.) ("GE-EH", prod. of Kao) | IOB=1.05 | | | | | | 0.61 | 0.73 | 0.74 | 0.71 |
| | POE(8) diisostearate ("EMALEX® 400di-IS", prod. of Nihon Emulsion) | IOB=0.63 | | | | | | | | | |
| | POE(6) diisostearate ("EMALEX® 300di-IS", prod. of Nihon Emulsion) | IOB=0.53 | | | | | | | | | |
| | POE(6) sorbitan monolaurate ("RHEODOL® SUPER TW-L106", prod. of Kao) | IOB=0.62 | | | | | | | | | |
| | Sorbitan Monostearate ("RHEODOL® SP-S10", product of Kao Corp.) | IOB=0.93 | | | | | | | | | |
| | Propylene glycol | | | | | | | | | | 0.50 |

1) I.V.: Inorganic Value.

EP 1 488 775 B1

Table 3 (Cont'd)

| | | Example | | | | | | | | |
|---|---|---|---|---|---|---|---|---|---|---|
| | | 12 | 13 | 14 | 15 | 16 | 17 | 18 | 19 | 20 |
| External appearance (25°C) | | Clear | Clear | Clear | Clear | Clear | Clear | Clear | Clear | Clear |
| Form | | Liq. | Liq. | Liq. | Liq. | Liq. | Liq. | Liq. | Liq. | Liq. |
| Make removal (oil-based) | | A | A | A | A | A | A | A | A | A |
| Transmittance (%) when mixed with water | Mixed with 10 wt. parts of water | A | A | A | A | A | A | A | A | A |
| | Mixed with 50 wt. parts of water | B | C | C | A | C | B | C | B | B |
| Makeup removal when mixed with 50 parts by weight of water (oil-based) | | B | B | B | B | C | B | C | B | B |
| Makeup removal when mixed with 50 parts by weight of water (lipstick) | | B | B | B | B | C | B | C | B | B |
| Rinsing property | | A | B | A | A | B | A | A | A | A |
| Overall performance | | A | B | A | A | B | A | B | A | A |

## Table 4

| Ingredient (wt.%) | | | | Comparative Example | | | | | | |
|---|---|---|---|---|---|---|---|---|---|---|
| | | | 7 | 8 | 9 | 10 | 11 | 12 | 13 |
| A | Diglycerol monolaurate ("SY-GLYSTER®", prod. of Sakamoto Yakuhin Kogyo) | IOB=1.06 | | | | | | | |
| | Diglycerol monooleate ("Sunsoft® Q17-B", prod. of Taiyo Kagaku) | IOB=0.80 | | | | | | | |
| | Hexaglycerol monooleate ("Sunsoft® Q-17F", prod. of Taiyo Kagaku) | IOB=1.20 | | | | | | | |
| | Diglycerol monoisostearate ("COSMOL® 41", prod. of The Nisshin Oil Mills) | IOB=0.79 | | | | | | 3.86 | 4.46 |
| | POE(3.3) mono(C$_{9-11}$ alkyl) ether ("SOFTANOL® 33", prod. of Nippon Shokubai) | IOB=0.77 | | | | | | 5.76 | 6.65 |
| | Isostearyl pentaerythryl glyceryl ether ("LC-2", prod. of Kao) | IOB=0.92 | | | | | | 1.03 | 1.19 |
| B | Polyoxyethylene monolaurate ("EMANON® 1112HG", prod. of Kao) | IOB=1.34 | 9.78 | 8.72 | 15.00 | 14.00 | 14.00 | | |
| | POE(7) glyceryl mono(coconut fatty acid) ("UNIGLY® MK-207, prod. of NOF) | IOB=1.22 | | | | | | | |
| | POE(20) sorbitan monolaurate ("RHEODOL® Super TW-L120", prod. of Kao) | IOB=1.53 | | | | | | | |
| | Alkyl polyglucoside (C$_{9-11}$, saccharide condensation degree: 1.3, 40% aq. soln.; prod. of Kao) | IOB=1.84 | | | | | | | |
| C | Decamethyl cyclopentasiloxane ("Silicone SH245", prod. of Dow Corning Toray Silicone) | | 19.06 | 17.44 | 20.00 | 20.00 | 20.00 | 19.34 | 22.34 |
| | Isopropyl palmitate ("EXCEPARL® IPP", prod. of Kao) | C=19, I.V.=60[1] | 9.53 | 8.72 | 11.00 | 13.80 | 10.00 | | |
| | Liquid paraffin ("HICALL® K350", prod. of KANEDA) | C=35, I.V.=0 | | | 16.00 | 15.00 | 15.00 | 14.44 | 16.68 |
| | Liquid paraffin ("HICALL® K230", prod. of KANEDA) | C=24, I.V.=0 | 47.66 | 52.35 | 26.00 | 28.00 | 33.40 | 38.42 | 44.39 |
| D | Purified water | | 1.63 | 1.74 | | 2.20 | 1.50 | 2.03 | 2.34 |
| E | Isostearic acid (prod. of Nissan Chemical Industries) | IOB=0.56 | 1.48 | 2.31 | | 1.00 | | 0.98 | 1.13 |
| | Myristyl alcohol ("KALCOL® 4098", prod. of Kao) | IOB=0.36 | | | 6.00 | | | | |
| Others | Decaglycerol oleate ("Sunsoft® Q-17S", prod. of Taiyo Kagaku) | IOB=1.40 | | 8.72 | | | | | |
| | Diglycerol diisostearate ("COSMOL® 42", prod. of The Nisshin Oil Mills) | IOB=0.41 | 10.86 | | | | | | |
| | 2-Ethylhexyl glyceryl ether (89% aq. soln.) ("GE-EH", prod. of Kao) | IOB=1.05 | | | | | | 0.71 | 0.82 |
| | POE(8) diisostearate ("EMALEX® 400di-IS", prod. of Nihon Emulsion) | IOB=0.63 | | | | 6.00 | | | |
| | POE(6) diisostearate ("EMALEX® 300di-IS", prod. of Nihon Emulsion) | IOB=0.53 | | | | | 6.10 | | |
| | POE(6) sorbitan monolaurate ("RHEODOL® SUPER TW-L106", prod. of Kao) | IOB=0.62 | | | | | | 13.44 | |
| | Sorbitan Monostearate ("RHEODOL® SP-S10", product of Kao Corp.) | IOB=0.93 | | | 6.00 | | | | |
| | Propylene glycol | | | | | | | | |

1) I.V.: Inorganic Value.

EP 1 488 775 B1

Table 4 (Cont'd)

| | | Comparative Example | | | | | | |
|---|---|---|---|---|---|---|---|---|
| | | 7 | 8 | 9 | 10 | 11 | 12 | 13 |
| External appearance (25°C) | | Semi-clear | - | Semi-clear | Clear | Clear | Clear | Clear |
| Form | | Liq. | - | Liq. | Liq. | Liq. | Liq. | Liq. |
| Make removal (oil-based) | | A | - | A | A | A | A | A |
| Transmittance (%) when mixed with water | Mixed with 10 wt. parts of water | C | - | B | D | C | D | D |
| | Mixed with 50 wt. parts of water | D | - | D | D | D | D | D |
| Makeup removal when mixed with 50 parts by weight of water (oil-based) | | D | - | D | E | E | B | B |
| Makeup removal when mixed with 50 parts by weight of water (lipstick) | | B | - | B | B | B | D | D |
| Rinsing property | | C | - | B | B | B | D | D |
| Overall performance | | D | - | D | D | D | D | D |

EP 1 488 775 B1

Table 5

| Ingredient (wt.%) | | Example | | | | | | | | | Comparative Example | | | | |
|---|---|---|---|---|---|---|---|---|---|---|---|---|---|---|---|
| | | 21 | 22 | 23 | 24 | 25 | 26 | 27 | 28 | 29 | 14 | 15 | 16 | 17 | 18 |
| A | Safflower monoglyceride ("Sunsoft® No. 8090)", prod. of Taiyo Kagaku)* | | | | | | 3.24 | | | | | | | | |
| | Glycerol oleate ("EXCEL® O-95R", prod. of Kao) | 1.30 | 1.40 | 1.40 | 1,40 | 1.26 | | 1.90 | 1.11 | 2.59 | | | | | |
| | Diglycerol monoisostearate ("COSMOL® 41V", prod. of The Nisshin Oil Mills) | 2.60 | 2.50 | 2.50 | 2.50 | 2.52 | | 2.00 | 2.76 | 5.43 | | | | 3.86 | 4.46 |
| | Diglycerol monooleate ("Sunsoft® Q17-B", prod. of Taiyo Kagaku) | | | | | | 3.23 | | | | | | | | |
| | POE(3.3) mono(C$_{9-11}$ alkyl) ether ("SOFTANOL® 33", prod. of Nippon Shokubai) | 3.70 | 2.40 | 3.60 | 2.40 | 4.85 | | 3.00 | 4.12 | | | | | 5.76 | 6.65 |
| | Isostearyl pentaerythryl glyceryl ether ("LC-2", prod. of Kao) | 1.00 | 1.05 | 1.05 | 1.05 | 0.97 | | 1.05 | 1.05 | | | | | 1.03 | 1.19 |
| B | Alkyl polyglucoside (C$_{9-11}$, saccharide condensation degree: 1.3; prod. of Kao) | 2.60 | 2.00 | 3.24 | 3.12 | 3.10 | | 2.90 | | | | | | | |
| | POE(20) sorbitan monolaurate ("RHEODOL® Super TW-L120", prod. of Kao) | | | | | | | 4.10 | 5.87 | | | | | | |
| | POE(7) glyceryl coconut fatty acid ("UNIGLY® MK-207, prod. of NOF) | | | | | | 11.58 | | | | | | | | |
| | Polyoxyethylene glyceryl monolaurate ("EMANON® 1112HG", prod. of Kao) | 6.20 | 9.75 | 8.05 | 8.20 | 7.59 | 3.04 | | 4.20 | 12.90 | 15.00 | 14.00 | 14.00 | | |
| C | Decamethyl cyclopentasiloxane ("Silicone SH245", prod. of Dow Corning Toray Silicone) | 18.50 | 18.00 | 18.00 | 18.00 | 17.93 | 5.69 | 20.00 | 19.85 | 19.10 | 20.00 | 20.00 | 20.00 | 19.34 | 22.34 |
| | Isopropyl palmitate ("EXCEPARL® IPP", prod. of Kao) | 5.00 | 4.70 | 4.70 | 4.70 | 4.85 | 2.85 | 6.00 | 1.74 | 7.20 | 11.00 | 13.80 | 10.00 | | |
| | Isotridecyl isononanoate ("SALACOS® 913", prod. of The Nisshin Oil Mills) | | | | | | 21.44 | | | | | | | | |
| | Liquid paraffin ("HICALL® 350", prod. of KANEDA) | 36.00 | 34.40 | 35.10 | 35.75 | 34.88 | | 35.80 | 38.65 | 13.68 | 16.00 | 15.00 | 15.00 | 14.43 | 16.68 |
| | Liquid paraffin ("HICALL® 230", prod. of KANEDA) | 16.00 | 15.50 | 15.50 | 15.50 | 14.54 | 46.36 | 15.96 | 15.14 | 34.30 | 26.00 | 28.00 | 33.40 | 38.42 | 44.39 |

* Contained 60 to 80 wt.% of Glycerol monolinoleate

Table 5 (Cont'd)

| | Ingredient (wt.%) | Example | | | | | | | | | Comparative Example | | | | |
|---|---|---|---|---|---|---|---|---|---|---|---|---|---|---|---|
| | | 21 | 22 | 23 | 24 | 25 | 26 | 27 | 28 | 29 | 14 | 15 | 16 | 17 | 18 |
| D | Purified water | 4.85 | 5.60 | 4.86 | 4.68 | 4.65 | 1.18 | 4.34 | 3.30 | 4.80 | | 2.20 | 1.50 | 2.03 | 2.34 |
| E | Isostearic acid (prod. of Nissan Chemical Industries) | 1.50 | 1.30 | 0.60 | 0.60 | 2.13 | 0.46 | 1.20 | 0.91 | | | 1.00 | | 0.98 | 1.13 |
| | Myristyl alcohol ("KALCOL® 4098", prod. of Kao) | 0.75 | 0.80 | 0.80 | 0.80 | | 0.93 | 1.00 | 0.57 | | 6.00 | | | | |
| | Cetyl alcohol ("KALCOL® 60", prod. of Kao) | | | 0.60 | | | | | | | | | | | |
| | Oleyl alcohol ("Oleyl Alcohol #1500", prod. of Kyowa Yushi Kogyo) | | | | 1.30 | | | | | | | | | | |
| Others | Isostearyl glyceryl ether ("GE-IS®", prod. of Kao) | | 0.50 | | | | | | | | | | | | |
| | Dibutylhydroxytoluene | | 0.10 | | | | | | | | | | | | |
| | 2-Ethylhexyl glyceryl ether (89% aq. soln.) ("GE-EH", prod. of Kao) | | | | | 0.73 | | 0.75 | 0.73 | | | | | 0.71 | 0.82 |
| | POE(8) diisostearate ("EMALEX® 400di-IS", prod. of Nihon Emulsion) | | | | | | | | | | | 6.00 | | | |
| | POE(6) diisostearate ("EMALEX® 300di-IS", prod. of Nihon Emulsion) | | | | | | | | | | | | 6.10 | | |
| | POE(6) sorbitan monolaurate ("RHEODOL SUPER® TW-L106", prod. of Kao) | | | | | | | | | | | | | 13.44 | |
| | Sorbitan Monostearate ("RHEODOL® SP-S10", product of Kao Corp.) | | | | | | | | | | 6.00 | | | | |

EP 1 488 775 B1

Table 6

| | | Example | | | | | | | | | Comparative Example | | | | |
|---|---|---|---|---|---|---|---|---|---|---|---|---|---|---|---|
| | | 21 | 22 | 23 | 24 | 25 | 26 | 27 | 28 | 29 | 14 | 15 | 16 | 17 | 18 |
| External appearance (25°C) | | Clear | Clear | Clear | Clear | Clear | Clear | Clear | Clear | Clear | Semi-clear | Clear | Clear | Clear | Clear |
| Form | | Liq. | Liq. | Liq. | Liq. | Liq. | Liq. | Liq. | Liq. | Liq. | Liq. | Liq. | Liq. | Liq. | Liq. |
| Make removal (oil-based) | | A | A | A | A | A | A | A | A | A | A | A | A | A | A |
| Transmittance (%) when mixed with water | Mixed with 10 wt. parts of water | A | A | A | A | C | A | A | A | A | B | D | C | D | D |
| | of water | B | B | B | B | C | B | B | C | C | D | D | D | D | D |
| Makeup removal when mixed with 50 parts by weight of water (oil-based) | | B | B | B | B | B | B | B | B | B | D | E | E | B | B |
| Makeup removal when mixed with 50 parts by weight of water (lipstick) | | B | B | B | B | B | B | C | C | B | B | B | B | D | D |
| Rinsing property | | A | A | A | A | A | B | A | A | B | A | B | B | D | D |
| Overall performance | | A | A | A | B | B | B | A | B | B | D | D | D | D | D |

EP 1 488 775 B1

Examples 30-33 & Comparative Examples 19-27

Cleansing preparations of the formulations presented in Table 7 and Table 9 were produced as in Examples 1-29, and the resultant cleansing preparations were similarly ranked in external appearance, form, makeup removal, transmittance and makeup removal when mixed with water, rinsing property, and overall performance. Further, the viscosities of the cleansing preparations were measured by the below-described method. The results are presented in Table 8 and Table 10.

(Production procedure)

The viscosity of each cleansing preparation was measured at 25°C by a Brookfield rotational viscometer (Rotor No. 2, 30 rpm). When the pointer shows a value over the highest limit , Rotor No. 3 or No. 4 was used as needed

Table 7

| Ingredient (wt.%) | | | Example | | Comparative Example | | | | |
|---|---|---|---|---|---|---|---|---|---|
| | | | 30 | 31 | 19 | 20 | 21 | 22 | 23 |
| A | Diglycerol monolaurate ("SY-GLYSTER®", prod. of Sakamoto Yakuhin Kogyo) | IOB=1.06 | 5.00 | | | | | | |
| | Diglycerol monoisostearate ("COSMOL® 41", prod. of The Nisshin Oil Mills) | IOB=0.79 | | 3.86 | | | | | 2.97 |
| | Diglycerol monooleate ("Sunsoft® Q17-B", prod. of Taiyo Kagaku) | IOB=0.80 | | | | 12.20 | | | |
| | POE(3.3) mono(C$_{9-11}$ alkyl) ether ("SOFTANOL® 33", prod. of Nippon Shokubai) | IOB=0.77 | | 5.69 | | | | | 4.38 |
| | Isostearyl pentaerythryl glyceryl ether ("LC-2", prod. of Kao) | IOB=0.92 | | 1.00 | | | | | 0.77 |
| B | Polyoxyethylene monolaurate ("EMANON® 1112HG", prod. of Kao) | IOB=1.34 | 11.00 | 6.27 | | | | | 4.82 |
| | POE(20) octyl dodecyl ether ("EMALEX® OD-20", prod. of Nihon Emulsion) | IOB=1.30 | | | | | 15.00 | 15.00 | |
| | Alkyl polyglucoside (C$_{9-11}$, saccharide condensation degree: 1.3, 40% aq. soln.; prod. of Kao) | IOB=1.84 | | 6.27 | | | | | 4.82 |
| | POE(20) sorbitan monooleate ("RHEODOL® Super TW-0120", prod. of Kao) | IOB=1.39 | | | | 7.80 | | | |
| C | Decamethyl cyclopentasiloxane ("Silicone SH245", prod. of Dow Corning Toray Silicone) | | 21.50 | 19.29 | | | | | 14.84 |
| | Liquid paraffin ("HICALL® K350", prod. of KANEDA) | C=35, I.V.=0 [1] | 15.80 | 15.43 | | | | | 11.87 |
| | Liquid paraffin ("HICALL® K230", prod. of KANEDA) | C=24, I.V.=0 | 39.00 | 40.50 | | | | | 31.15 |
| | Glyceryl tri(2-ethylhexanoate) ("EXCEPARL® TGO", prod. of Kao) | C=27, I.V.=180 | | | | | 27.50 | 27.50 | |
| | Liquid isoparaffin ("PARMLEAM® EX", prod. of NOF) | C=21, I.V.=0 | | | | | 27.50 | 27.50 | |
| | Squalane (product of Kishimoto Special Liver Oil Co., Ltd.) | C=30, I.V.=0 | | | 11.00 | 11.00 | | | |
| | Glyceryl tri-caprylate/caprate ("COCONAD® MT", prod. of Kao) | C=28, I.V.=180 | | | 11.00 | 11.00 | | | |
| D | Purified water | | 3.00 | | 29.00 | 42.00 | 6.50 | 6.50 | 15.39 |
| E | Isostearic acid (prod. of Nissan Chemical Industries) | IOB=0.56 | 0.70 | 0.97 | | | | | 0.75 |
| | Myristyl alcohol ("KALCOL® 4098", prod. of Kao) | IOB=0.36 | 4.00 | | | | | | |
| Others | Decaglycerol monolaurate ("SY-GLYSTER®", prod. of Sakamoto Yakuhin Kogyo) | IOB=1.60 | | | 15.00 | | | | |
| | POE(3) oleyl ether ("EMALEX® 503", prod. of Nihon Emulsion) | IOB=0.57 | | | 15.00 | | | | |
| | POE(10) hydrogenated castor oil ("EMALEX® HC-10", prod. of Nihon Emulsion) | IOB=0.69 | | | | | 6.00 | | |
| | POE(20) hydrogenated castor oil ("EMALEX® HC-20", prod. of Nihon Emulsion) | IOB=0.94 | | | | | | 6.00 | |
| | 2-Ethylhexyl glyceryl ether, 89% aq. soln. ("GE-EH", prod. of Kao) | IOB=1.05 | | 0.72 | | | | | 0.55 |
| | 1,3-Butylene glycol | | | | 19.00 | 16.00 | | | 7.69 |
| | Glycerol | | | | | | 17.50 | 17.50 | |

1) I.V.: Inorganic Value.

EP 1 488 775 B1

Table 8

| | | Example | | Comparative Example | | | | |
|---|---|---|---|---|---|---|---|---|
| | | 30 | 31 | 19 | 20 | 21 | 22 | 23 |
| External appearance (25°C) | | Clear | Clear | Clear | Semi-clear | Clear | Clear | Semi-clear |
| Form | | Liq. | Liq. | Liq. | Loose gel | Liq. | Liq. | Liq. |
| Viscosity (mPa·s) | | 40 | 40 | 130 | 10800 | 520 | 810 | 45 |
| Make-up removal (oil-based) | | A | A | C | D | D | D | C |
| When mixed with 50 parts by weight of water | Transmittance (%) | B | B | D | C | D | D | D |
| | Viscosity (mPa·s) | 45 | 40 | 800 | 4600 | 560* | 280* | 110 |
| | Makeup removal (oil-based) | B | B | D | E | E | E | E |
| | Makeup removal (lipstick) | B | B | D | E | E | E | E |
| Rinsing property | | A | A | C | B | B | B | B |
| Overall performance | | A | A | D | D | D | D | D |
| * A substantial viscosity increase when 10 parts by weight of water were added. | | | | | | | | |

## Table 9

| Ingredient (wt.%) | | Example | | Comparative Example | | | |
|---|---|---|---|---|---|---|---|
| | | 32 | 33 | 24 | 25 | 26 | 27 |
| A | Glycerol monooleate ("EXCEL® O-95R", prod. of Kao) | 1.40 | | | | | |
| | Sufflower monoglyceride ("Sunsoft® No. 8090, prod. of Taiyo Kagaku)* | | 3.09 | | | | |
| | Diglycerol monoisostearate ("COSMOL® 41V", prod. of The Nisshin Oil Mills) | 2.50 | | | | | |
| | Diglycerol monooleate ("Sunsoft® Q17-B", prod. of Taiyo Kagaku) | | 3.08 | | 12.20 | | |
| | POE(3.3) mono(C$_{9-11}$ alkyl) ether ("SOFTANOL® 33", prod. of Nippon Shokubai) | 3.70 | | | | | |
| | Isostearyl pentaerythryl glyceryl ether ("LC-2", prod. of Kao) | 1.05 | | | | | |
| B | Polyoxyethylene monolaurate ("EMANON® 1112HG", prod. of Kao) | 7.50 | 2.90 | | | | |
| | POE(20) octyl dodecyl ether ("EMALEX® OD-20", prod. of Nihon Emulsion) | | | | | 15.00 | 15.00 |
| | POE(7) glyceryl coconut fatty acid ("UNIGLY® MK-207", prod. of NOF) | | 11.05 | | | | |
| | Alkyl polyglucoside (C$_{9-11}$, saccharide condensation degree: 1.3; prod. of Kao) | 3.20 | | | | | |
| | POE(20) sorbitan monooleate ("RHEODOL® Super TW-0120", prod. of Kao) | | | | 7.80 | | |
| C | Decamethyl cyclopentasiloxane ("Silicone SH245", prod. of Dow Corning Toray Silicone) | 18.00 | 5.43 | | | | |
| | Isopropyl palmitate ("EXCEPARL® IPP", prod. of Kao) | 4.50 | 2.72 | | | | |
| | Liquid paraffin ("HICALL® 350", prod. of KANEDA) | 15.50 | | | | | |
| | Liquid paraffin ("HICALL® 230", prod. of KANEDA) | 35.04 | 44.24 | | | | |
| | Isotridecyl isononanoate ("SALACOS® 913", prod. of The Nisshin Oil Mills) | | 20.44 | | | | |
| | 2-Ethylhexanoic acid triglyceride ("EXCEPARL® TGO", prod. of Kao) | | | | | 27.50 | 27.50 |
| | Liquid isoparaffin ("PARMLEAM® EX", prod. of NOF) | | | | | 27.50 | 27.50 |
| | Squalane (product of Kishimoto Special Liver Oil) | | | 11.00 | 11.00 | | |
| | Glyceryl tri-caprylate/caprate ("COCONAD® MT", prod. of Kao) | | | 11.00 | 11.00 | | |
| D | Purified water | 4.80 | 5.72 | 29.00 | 42.00 | 6.50 | 6.50 |
| E | Isostearic acid (prod. of Nissan Chemical Industries) | 1.30 | 0.44 | | | | |
| | Myristyl alcohol ("KALCOL® 4098", prod. of Kao) | 0.81 | 0.89 | | | | |
| Others | Decaglycerol monolaurate ("SY-GLYSTER®", prod. of Sakamoto Yakuhin Kogyo) | | | 15.00 | | | |
| | POE(3) oleyl ether ("EMALEX® 503", prod. of Nihon Emulsion) | | | 15.00 | | | |
| | POE(10) hydrogenated castor oil ("EMALEX® HC-10", prod. of Nihon Emulsion) | | | | | 6.00 | |
| | POE(20) hydrogenated castor oil ("EMALEX® HC-20", prod. of Nihon Emulsion) | | | | | | 6.00 |
| | 2-Ethylhexyl glyceryl ether, 89% aq. soln. ("GE-EH®", prod. of Kao) | 0.70 | | | | | |
| | 1,3-Butylene glycol | | | 19.00 | 16.00 | | |
| | Glycerol | | | | | 17.50 | 17.50 |

* Contained 60 to 80 wt.% of Glycerol monolinoleate

EP 1 488 775 B1

Table 10

| | | Example | | Comparative Example | | | |
|---|---|---|---|---|---|---|---|
| | | 32 | 33 | 24 | 25 | 26 | 27 |
| External appearance (25°C) | | Clear | Clear | Clear | Semi-clear | Clear | Clear |
| Form | | Liq. | Liq. | Liq. | Loose gel | Liq. | Liq. |
| Viscosity (mPa·s) | | 38 | 30 | 130 | 10800 | 520 | 810 |
| Make removal (oil-based) | | A | A | C | D | D | D |
| When mixed with 50 parts by weight of water | Transmittance (%) | B | B | D | C | D | D |
| | Viscosity (mPa·s) | 40 | 60 | 800 | 4600 | 560* | 280* |
| | Makeup removal (oil-based) | B | B | D | E | E | E |
| | Makeup removal (lipstick) | B | B | D | E | E | E |
| Rinsing property | | A | B | C | B | B | B |
| Overall performance | | A | A | D | D | D | D |
| * A remarkable viscosity increase when 10 parts by weight of water were added. | | | | | | | |

Examples 34-39

Cleansing preparations of the formulations presented in Table 11 were produced as in Examples 1-29, and the resultant cleansing preparations were similarly ranked in external appearance, form, makeup removal, transmittance and make-up removal when mixed with water, rinsing property, and overall performance. Further, the cleansing preparations were measured on its storage stability by the below-describedmethod. The results are presented in Table 11.

(Storage stability)

Each cleansing preparation (50 g) was stored at 0°C and 5°C. Its external appearance was visually ranked one week later, and its storage stability was indicated in accordance with the following standards.

A: Clear without any precipitate.
B: A precipitate is settled, or the whole cleansing preparation is turbid by a precipitate.

Table 11

| Ingredient (wt.%) | | Example | | | | | |
|---|---|---|---|---|---|---|---|
| | | 34 | 35 | 36 | 37 | 38 | 39 |
| A | Glycerol oleate ("EXCEL® O-95R", prod. of Kao) | 2.40 | 1.88 | 1.69 | 1.40 | 1.03 | 0.85 |
| | Diglycerol monoisostearate ("COSMOL® 41V", prod. of The Nisshin Oil Mills) | 1.50 | 2.04 | 2.22 | 2.50 | 2.86 | 3.04 |
| | POE(3.3) mono($C_{9-11}$ alkyl) ether ("SOFTANOL® 33", prod. of Nippon Shokubai) | 2.50 | 3.02 | 3.29 | 3.70 | 4.22 | 4.49 |
| | Isostearyl pentaerythryl glyceryl ether ("LC-2", prod. of Kao) | 1.50 | 1.07 | 1.06 | 1.05 | 1.04 | 1.04 |

(continued)

| Ingredient (wt.%) | | Example | | | | | |
|---|---|---|---|---|---|---|---|
| | | 34 | 35 | 36 | 37 | 38 | 39 |
| B | Alkyl polyglucoside (C$_{9-11}$, saccharide condensation degree: 1.3; prod. of Kao) | 5.10 | 5.19 | 5.05 | 4.80 | 4.55 | 4.41 |
| | Polyethylene glycol monolaurate ("EMANON® 1112HG", prod. of Kao) | 8.30 | 7.13 | 7.04 | 7.50 | 6.74 | 6.66 |
| C | Decamethyl cyclopentasiloxane ("Silicone SH245", prod. of Dow Corning Toray Silicone) | 18.00 | 18.34 | 18.44 | 18.00 | 18.78 | 18.88 |
| | Isopropyl palmitate ("EXCEPARL® IPP", prod. of Kao) | 6.00 | 5.90 | 5.31 | 4.50 | 3.25 | 2.66 |
| | Liquid paraffin ("HICALL® 350", prod. of KANEDA) | 33.00 | 33.59 | 34.28 | 35.04 | 36.72 | 37.37 |
| | Liquid paraffin ("HICALL® 230", prod. of KANEDA) | 15.00 | 15.28 | 15.29 | 15.50 | 15.32 | 15.33 |
| D | Purified water | 3.40 | 3.46 | 3.37 | 3.20 | 3.03 | 2.94 |
| E | Isostearic acid (prod. of Nissan Chemical Industries) | 1.20 | 1.28 | 1.25 | 1.30 | 1.14 | 1.11 |
| | Myristyl alcohol ("KALCOL® 4098", prod. of Kao) | 1.10 | 1.06 | 0.95 | 0.81 | 0.58 | 0.48 |
| | 2-Ethylhexyl glyceryl ether, 89% aq. soln. ("GE-EH", prod. of Kao) | 1.00 | 0.76 | 0.76 | 0.70 | 0.74 | 0.74 |
| External appearance (25°C) | | Clear | Clear | Clear | Clear | Clear | Clear |
| Form | | Liq. | Liq. | Liq. | Liq. | Liq. | Liq. |
| Makeup removal (oil-based) | | A | A | A | A | A | A |
| Transmittance (%) when mixed with 50 parts by weight of water | | B | B | B | B | B | B |
| Makeup removal (oil-based) when mixed with 50 parts by weight of water | | B | B | B | B | B | B |
| Rinsing property | | A | A | A | A | A | B |
| Storage stability | 0°C | A | A | A | A | A | A |
| | 5°C | A | A | A | A | A | A |

Examples 40-49 & Comparative Examples 28-32

Cleansing preparations of the formulations presented in Table 12 and Table 13 were produced as in Examples 1-29. The resultant cleansing preparations were similarly ranked in external appearance and form as in Examples 1-29, and in addition, they were also ranked in a temperature range in which no turbidity took place even when mixed with water, external appearance and removal of oily dirts when mixed with water, external appearance and massage property when applied to the wet skin, and rinsability. The results are also presented in Table 12 and Table 13.

(Production procedure)

The ingredients (A), (B), (C), (D) and (E) were stirred into a uniform mixture to obtain a liquid, oil-based preparation.

(Ranking methods)

(1) Turbidity-free temperature range (25 to 70°C) in a form mixed with 100 parts by weight of water

Water (3 g) and an oil-based preparation (3 g) were placed in a 20-mL screw capped tube. After the contents were heated to 70°C while shaking, they were slowly cooled to 25°C while shaking. Temperatures were recorded by digital thermometer at which the following states were observed: the wall of the glass bottle has become transparently visible from its initially observed opacity, and the wall of the glass bottle has become opaque from its transparent appearance. Those temperatures were recorded as a turbidity-free temperature range in a form mixed with 100 parts by weight of water.

(2) External appearance during mixing with 100 parts by weight of water at 30°C

With respect to each oil-based preparation, the transmittance was measured at a wavelength of 530 nm by a turbiditymeter (digital colorimeter "mini photo® 5"; equipped with a glass cell of 10 mm in diameter; manufactured by Sanshin Kogyo Co., Ltd.) in the course of addition of water, whereby 10 parts each by weight of water was added stepwise, up to 100 parts by weight in total of 100 parts by weight of the preparation, while using purified water as a reference. The transmittance was ranked in accordance with the below-described standards by comparing it with the transmittance of an aqueous suspension of titanium oxide ("Titanium Oxide JA-C", particle size: 0.1 to 0.5 $\mu$m or so; product of Tayca Corporation).

A: Constantly higher than the transmittance of a 0.02 wt.% aqueous suspension of titanium oxide in the course of mixing of water up to 100 parts by weight.
B: Became lower than the transmittance of a 0.02 wt.% aqueous suspension of titanium oxide in the course of mixing with 50 to 100 parts by weight of water.
C: Became lower than the transmittance of a 0.02 wt.% aqueous suspension of titanium oxide in the course of mixing with 10 to 40 parts by weight of water.

(3) Removal of oil dirts with a preparation in a form mixed with 100 parts by weight of water

A lipstick ("Lipfinity® Color Liquid Base", product of Max Factor) was applied thinly over areas of 0.5 cm in diameter on the forearm, and was left for 15 minutes to dryness. About 25 mg of the cleansing preparations were placed on the areas, respectively, and each area was lightly massaged 30 times with a finger. The area was then rinsed with running water, and the result was visually observed as to how much of the lipstick was remaining at each of the areas. The observation results were ranked in accordance with the following standards.

A: Good removal of the lipstick
B: Poor removal of the makeup

(4) External appearance when applied to the wet skin

With respect to each liquid oil-based preparation, the preparation (2 g) was dispensed on the palms from which water had not been swished off subsequent to soaking of the palms with tepid water of about 36°C. The preparation was spread well by rubbing both hands together. Ranking was then performed as to the external appearance of the preparation spread over the palms.

A: The palms did not become white.
B: The palms looked white at some area.
C: The palms looked white in their entirety.

(5) Massage property when applied to the wet skin

With respect to each liquid, oil-based preparation, the preparation (2 g) was dispensed on the palms from which water had not been swished off subsequent to soaking of the palms with water. The preparation was spread well by rubbing both hands together. Organoleptic ranking was then performed as to slide feeling.

A: Smoothly slid well with good massage property.
B: Smoothly slid with good massage property.

C: Slide feeling was weak as sliding was too heavy or too light, and massage property was poor.

(6) Rinsability

With respect to each liquid, oil-based preparation, the preparation (2 g) was dispensed on the palms from which water had not been swished off subsequent to soaking of the palms with water. The preparation was spreadover the forearm. Upon rinsing it off with tepid water, ranking was performed as to its rinsability and the feeling of the skin after it was washed away.

A: Rinsed clean and refreshed.
B: Easily rinsable, but not refreshed.
C: A slimy touch remained on the skin even after rinsed for a long time, and hence, the preparation was hardly rinsable.

Table 12

| Ingredient (wt.%) | | IOB | Example | | | | | Comparative Example | | |
|---|---|---|---|---|---|---|---|---|---|---|
| | | | 40 | 41 | 42 | 43 | 44 | 28 | 29 | 30 |
| A | POE(12) diisostearate ("EMALEX® 600di-IS", prod. of Nihon Emulsion) | 0.80 | | | 8.2 | | | | | |
| | POE(30) sorbitol tetraoleate ("RHEODOL® 430", prod. of Kao) | 0.90 | 9.0 | | | 8.0 | | 15.0 | | 20.0 |
| | POE(8) glyceryl monoisostearate ("EMALEX® GWIS108", prod. of Nihon Emulsion) | 0.95 | | | | | 8.5 | | | |
| | POE(6) monoisostearate ("PEIS-6", prod. of Nihon Emulsion) | 0.91 | | 9.5 | | | | | | |
| B | POE(7) glyceryl coconut fatty acid ("UNIGLY® MK-207, prod. of NOF) | 1.22 | | | 17.0 | 14.0 | 11.0 | | | |
| | Polyethylene glycol monolaurate ("EMANON® 1112HG", prod. of Kao) | 1.34 | 14.0 | 15.0 | | | | 10.0 | 20.0 | |

(continued)

| Ingredient (wt.%) | | IOB | Example | | | | | Comparative Example | | |
|---|---|---|---|---|---|---|---|---|---|---|
| | | | 40 | 41 | 42 | 43 | 44 | 28 | 29 | 30 |
| C | Isotridecyl isononanoate ("SALACOS® 913", prod. of The Nisshin Oillio Group, Ltd.) | | | | 65.0 | | | | | |
| | Liquid paraffin ("HICALL® K230", prod. of KANEDA) | | 69.4 | 67.7 | | 69.5 | 73.0 | 74.5 | 72.0 | 73.0 |
| D | Purified water | | 3.6 | 3.0 | 5.0 | 0.5 | 0.5 | 0.5 | 3.0 | 2.0 |
| E | POE(6) diisostearate ("EMALEX® 300di-IS", prod. of Nihon Emulsion) | 0.53 | | | | 8.0 | | | | |
| | Myristyl alcohol ("KALCOL® 4098", prod. of Kao) | 0.36 | | | 1.8 | | 4.0 | | | |
| | Propylene glycol monolaurate ("EMALEX® PGML", product of Nihon Emulsion) | 0.53 | 4.0 | | | | | | 5.0 | |
| | diisostearate ("COSMOL® 42", prod. of The Nisshin Oil Mills) | 0.41 | | | | | 3.0 | | | 5.0 |
| | Diglycerol Isostearyl glyceryl ether ("GE-IS(U)", prod. of Kao) | 0.54 | | 4.8 | 3.0 | | | | | |
| mixed IOB (A+B+E) | | | 1.07 | 1.07 | 0.99 | 0.95 | 0.91 | 1.10 | 1.10 | 0.80 |
| (A)/(A+B) | | | 0.39 | 0.39 | 0.33 | 0.36 | 0.44 | 0.60 | - | - |
| External appearance (25°C) | | | Clear | Clear | Clear | Clear | Clear | Clear | Semi-clear | Clear |
| Form | | | Liq. | Liq. | Liq. | Liq. | Liq. | Liq. | Liq. | Liq. |
| Turbidity-free temperature range when mixed with 100 parts by weight of water (measured at 25 to 70°C) | | | 27-46 | 25-42 | 25-37 | 25-40 | 25-44 | 37-54 | None | None |
| External appearance until mixed at 30°C with 100 parts by weight of water | | | A | A | A | A | A | C | C | C |

(continued)

| Ingredient (wt.%) | IOB | Example | | | | | Comparative Example | | |
|---|---|---|---|---|---|---|---|---|---|
| | | 40 | 41 | 42 | 43 | 44 | 28 | 29 | 30 |
| Removal of oil dirts with the preparation in a form mixed at 30°C with 100 parts by weight of water | | A | A | A | A | A | B | B | A |
| External appearance when applied to the wet skin | | A | A | B | A | B | C | C | c |
| Massage feeling when applied to the wet skin | | A | A | B | A | B | C | C | C |
| Rinsability | | A | A | A | A | A | B | A | C |

Table 13

| Ingredient (wt.%) | | IOB | Example | | | | | Comparative Example | |
|---|---|---|---|---|---|---|---|---|---|
| | | | 45 | 46 | 47 | 48 | 49 | 31 | 32 |
| A | POE(30) sorbitol tetraoleate ("RHEODOL® 430", prod. of Kao) | 0.90 | 14.0 | 12.0 | 10.0 | 8.0 | 5.0 | 4.5 | 8.0 |
| B | Polyethylene glycol monolaurate ("EMANON® 1112HG", prod. of Kao) | 1.34 | 9.0 | 11.0 | 12.0 | 12.0 | 15.0 | 18.0 | 4.0 |
| C | Liquid paraffin ("HICALL® K230", prod. of KANEDA) | | 72.9 | 74.2 | 70.2 | 73.5 | 66.0 | 76.0 | 16.0 |
| D | Purified water | | 3.0 | 0 | 4.0 | 2.0 | 7.0 | 0 | 0 |
| E | Propylene glycol monolaurate ("EMALEX® PGML", product of Nihon Emulsion) | 0.53 | 1.1 | 2.8 | 3.8 | 4.5 | 7.0 | 1.5 | 12.0 |
| Mixed IOB (A+B+E) | | | 1.05 | 1.05 | 1.05 | 1.05 | 1.05 | 1.21 | 0.79 |
| (A)/(A+B) | | | 0.61 | 0.52 | 0.45 | 0.40 | 0.25 | 0.20 | 0.67 |
| External appearance (25°C) | | | Clear | Clear | Clear | Clear | Clear | Semi-clear | Clear |
| Form | | | Liq. | Liq. | Liq. | Liq. | Liq. | Liq. | Liq. |
| Turbidity-free temperature range when mixed with 100 parts by weight of water (measured at 25 to 70°) | | | 31-46 | 25-44 | 25-42 | 25-3A | 25-36 | None | None |
| External appearance until mixed at 30°C with 100 parts by weight of water | | | A | A | A | A | A | C | C |
| Removal of oil dirts with the preparation in a form mixed at 30°C with 100 parts by weight of water | | | A | A | A | A | A | B | A |

(continued)

| Ingredient (wt.%) | | IOB | Example | | | | | Comparative Example | |
|---|---|---|---|---|---|---|---|---|---|
| | | | 45 | 46 | 47 | 48 | 49 | 31 | 32 |
| External appearance when applied to the wet skin | Tepid water of about 36°C | | A | A | A | A | B | C | C |
| | Cold water of about 17°C | | A | A | A | A | B | C | C |
| Massage feeling when applied to the wet skin | | | A | A | A | A | B | C | C |
| Rinsability | | | B | B | A | A | A | A | C |

## Industrial Applicability

The cleansing preparation according to the present invention presents a clear and beautiful liquid form, and even when water is mixed in, retains a solubilized form and remains free of turbidity during use, and assures good sensation upon application without any reduction in cleansing power. Therefore, it can be applied to the wet skin, and is suited especially for use in the bath room.

## Claims

1. A clear liquid cleansing preparation comprising the following ingredients (A) to (E):

   (A) at least one nonionic surfactant selected from:

      (a) a monoglycerol fatty acid ester having a $C_{12-18}$ fatty acid residue,
      (b) a polyglycerol fatty acid ester having a $C_{12-18}$ fatty acid residue,
      (c) isostearyl pentaerythryl glyceryl ether, or
      (d) a liquid nonionic surfactant having polyalkylene glycol chains and exhibiting IOB value of from 0.75 to 1.05,

   (B) 5 to 30 wt.'% of a nonionic surfactant other than the first-mentioned nonionic surfactant (A), and exhibiting IOB value of at least 1.1,
   (C) 50 to 85 wt.% of a liquid oil ingredient,
   (D) at most 12 wt.% of water, and
   (E) 0.5 to 15 wt.% a nonionic surfactant, higher alcohol, higher fatty acid or glycerol derivative, each having IOB value not greater than 0.6,

   wherein said preparation is free of white turbidity when 50 parts by weight of water is added to 100 parts by weight of said preparation, and wherein the IOB of the mixture of the ingredients (A), (B) and (E) is from 0.8 to 1.2.

2. The cleansing preparation according to claim 1, wherein said nonionic surfactant (B) is at least one nonionic surfactant selected from a polyoxyethylene mono fatty acid ester, a polyoxyethylene glycerol fatty acid ester, a polyoxyethylene glyceryl mono fatty acid ester, an alkyl glucoside or a polyoxyethylene sorbitan fatty acid ester.

3. The cleansing preparation according to claim 1 or 2, which comprises from 1 to 15 wt.% of said ingredient (A).

4. The cleansing preparation according to any one of claims 1-3, wherein said monoglycerol fatty acid ester (a) in said ingredient (A) is selected from glycerol monooleate, glycerol monolinoleate or glycerol monoisostearate.

5. The cleansing preparation according to any one of claims 1-4, wherein said polyglycerol fatty acid ester (b) in said ingredient (A) is an ester of a $C_{12-18}$ fatty acid with a polyglycerol having a polymerization degree of from 2 to 6.

6. The cleansing preparation according to any one of claims 1-5, which comprises, as said ingredient (A), said monoglycerol fatty acid ester (a) and said polyglycerol fatty acid ester (b) in a range of from 20:80 to 80:20 (weight ratio).

**7.** The cleansing preparation according to any one of claims 1-6, wherein said liquid nonionic surfactant (d) in said ingredient (A) is selected from a polyoxyethylene fatty acid ester, a polyoxyethylene alkyl ether, a fatty acid polyoxyethylene alkyl ether, a polyoxyethylene sorbitan fatty acid ester, a polyoxyethylene sorbitol fatty acid ester, a polyoxyethylene glycerol fatty acid ester, a polyoxyethylene hydrogenated castor oil, or a polyoxyethylene-polyoxypropylene alkyl ether.

**8.** The cleansing preparation according to any one of claims 1-7, wherein a temperature at which said preparation takes a clear or semi-clear form when up to 100 parts by weight of water is added to and mixed with 100 parts by weight of said preparation lies within a range of from 28 to 32°C.

**9.** A cleansing preparation comprising:

(A) a liquid nonionic surfactant having a polyalkylene glycol chain and IOB value of from 0.75 to 1.05,
(B) 5 to 30 wt.% of a nonionic surfactant having IOB value of at least 1.1,
(C) 50 to 85 wt.% of a liquid oil ingredient,
(D) at most 12 wt.% of water, and
(E) 0.5 to 15 wt.% of a nonionic surfactant, higher alcohol, higher fatty acid or glycerol derivative, each having IOB value of 0.6 or lower,

wherein a temperature at which said preparation takes a clear or semi-clear form when up to 100 parts by weight of water is added to and mixed with 100 parts by weight of said preparation lies within a range of from 28 to 32°C, wherein the IOB of the mixture of the ingredients (A), (B) and (E) is from 0.8 to 1.2.

**Patentansprüche**

**1.** Klares flüssiges Reinigungspräparat, umfassend die folgenden Bestandteile (A) bis (E):

(A) zumindest ein nichtionisches Tensid, ausgewählt aus:

(a) einem Monoglycerylfettsäureester mit einem $C_{12-18}$-Fettsäurerest,
(b) einem Polyglycerylfettsäureester mit einem $C_{12-18}$-Fettsäurerest,
(c) Isostearylpentaerythrylglycerylether oder
(d) einem flüssigen nichtionischen Tensid mit Polyalkylenglycolketten und mit einem IOB-Wert von 0,75 bis 1,05,

(B) 5 bis 30 Gew.-% eines anderen nichtionischen Tensides als dem zuerst genannten nichtionischen Tensid (A) und mit einem IOB-Wert von wenigstens 1,1,
(C) 50 bis 85 Gew.-% eines flüssigen Ölbestandteils,
(D) maximal 12 Gew.-% Wasser, und
(E) 0,5 bis 15 Gew.-% eines nichtionischen Tensides, höheren Alkohols, höheren Fettsäure oder Glycerinderivates, jeweils mit einem IOB-Wert von nicht mehr als 0,6,

worin das Präparat frei von weißer Trübheit ist, wenn 50 Gew.-Teile Wasser zu 100 Gew.-Teilen des Präparates gegeben werden, und worin der IOB der Mischung der Bestandteile (A), (B) und (E) von 0,8 bis 1,2 ist.

**2.** Reinigungspräparat nach Anspruch 1, worin das nichtionische Tensid (B) zumindest ein nichtionisches Tensid ist, ausgewählt aus einem Polyoxyethylenmonofettsäureester, Polyoxyethylenglycerinfettsäureester, Polyoxyethylenglycerylmonofettsäureester, Alkylglucosid oder Polyoxyethylensorbitanfettsäureester.

**3.** Reinigungspräparat nach Anspruch 1 oder 2, umfassend von 1 bis 15 Gew.-% des Bestandteils (A).

**4.** Reinigungspräparat nach einem der Ansprüche 1 bis 3, worin der Monoglycerylfettsäureester (a) in dem Bestandteil (A) ausgewählt ist aus Glycerylmonooleat, Glycerylmonolinoleat oder Glycerylmonoisostearat.

**5.** Reinigungspräparat nach einem der Ansprüche 1 bis 4, worin der Polyglycerylfettsäureester (b) in dem Bestandteil (A) ein Ester einer $C_{12-18}$-Fettsäure mit einem Polyglycerin mit einem Polymerisationsgrad von 2 bis 6 ist.

**6.** Reinigungspräparat nach einem der Ansprüche 1 bis 5, umfassend als Bestandteil (A) den Monoglycerylfettsäureester (a) und den Polyglycerylfettsäureester (b) in einem Bereich von 20:80 bis 80:20 (Gewichtsverhältnis).

**7.** Reinigungspräparat nach einem der Ansprüche 1 bis 6, worin das flüssige nichtionische Tensid (d) in dem Bestandteil (A) ausgewählt ist aus einem Polyoxyethylenfettsäureester, Polyoxyethylenalkylether, Fettsäurepolyoxyethylenalkylether, Polyoxyethylensorbitanfettsäureester, Polyoxyethylensorbitolfettsäureester, Polyoxyethylenglycerinfettsäureester, Polyoxyethylenhydrierten Castoröl oder Polyoxyethylen-Polyoxypropylenalkylether.

**8.** Reinigungspräparat nach einem der Ansprüche 1 bis 7, worin eine Temperatur, bei der das Präparat eine klare oder halbklare Form annimmt, wenn bis zu 100 Gew.-Teile Wasser zugegeben und mit 100 Gew.-Teilen des Präparates vermischt sind, innerhalb eines Bereiches von 28 bis 32°C liegt.

**9.** Reinigungspräparat, umfassend:

(A) ein flüssiges nichtionisches Tensid mit einer Polyalkylenglycolkette und einem IOB-Wert von 0,75 bis 1,05,
(B) 5 bis 30 Gew.-% eines nichtionischen Tensides mit einem IOB-Wert von wenigstens 1,1,
(C) 50 bis 85 Gew.-% eines flüssigen Ölbestandteils,
(D) maximal 12 Gew.-% Wasser, und
(E) 0,5 bis 15 Gew.-% eines nichtionischen Tensides, höheren Alkohols, höherer Fettsäure oder Glycerinderivates, die jeweils einen IOB-Wert von 0,6 oder weniger haben,

worin eine Temperatur, bei der das Präparat eine klare oder halbklare Form annimmt, wenn bis zu 100 Gew.-Teile Wasser zugegeben und mit 100 Gew.-Teilen des Präparates gemischt werden, innerhalb eines Bereiches von 28 bis 32°C liegt, worin der IOB der Mischung der Bestandteile (A), (B) und (E) von 0,8 bis 1,2 ist.

**Revendications**

**1.** Préparation démaquillante liquide transparente comprenant les ingrédients (A) à (E) suivants :

(A) au moins un tensioactif non ionique choisi parmi :

(a) un ester d'acide gras de monoglycérol comprenant un résidu d'acide gras en $C_{12}$-$C_{18}$,
(b) un ester d'acide gras de polyglycérol comprenant un résidu d'acide gras en $C_{12}$-$C_{18}$,
(c) un éther isostéaryl pentaérythryl glycéryle, ou
(d) un tensioactif non ionique liquide comprenant des chaînes de polyalkylène glycol et présentant une valeur d'IOB allant de 0,75 à 1,05,

(B) 5 à 30 % en poids d'un tensioactif non ionique autre que le premier tensioactif non ionique mentionné (A), et présentant une valeur d'IOB d'au moins 1,1,
(C) 50 à 85 % en poids d'un ingrédient huileux liquide,
(D) 12 % en poids d'eau maximum, et
(E) 0,5 à 15 % en poids d'un tensioactif non ionique, d'un alcool supérieur, d'un acide gras supérieur ou d'un dérivé de glycérol, chacun ayant une valeur d'IOB inférieure ou égale à 0,6,

dans laquelle ladite préparation est exempte de turbidité blanche lorsque 50 parts en poids d'eau sont ajoutées à 100 parts en poids de ladite préparation, et dans laquelle l'IOB du mélange des ingrédients (A), (B) et (E) va de 0,8 à 1,2.

**2.** Préparation démaquillante selon la revendication 1, dans laquelle ledit tensioactif non ionique (B) est au moins un tensioactif non ionique choisi parmi un monoester d'acide gras de polyoxyéthylène, un ester d'acide gras de polyoxyéthylène glycérol, un monoester d'acide gras de polyoxyéthylène glycéryle, un glycoside d'alkyle ou un ester d'acide gras de polyoxyéthylène sorbitane.

**3.** Préparation démaquillante selon la revendication 1 ou 2, qui comprend 1 à 15 % en poids dudit ingrédient (A).

**4.** Préparation démaquillante selon l'une quelconque des revendications 1 à 3, dans laquelle ledit ester d'acide gras de monoglycérol (a) dans ledit ingrédient (A) est choisi parmi le monooléate de glycérol, le monolinoléate de glycérol ou le monoisostéarate de glycérol.

5. Préparation démaquillante selon l'une quelconque des revendications 1 à 4, dans laquelle ledit ester d'acide gras de polyglycérol (b) dans ledit ingrédient (A) est un ester d'acide gras en $C_{12}$-$C_{18}$ avec un polyglycérol ayant un degré de polymérisation de 2 à 6.

6. Préparation démaquillante selon l'une quelconque des revendications 1 à 5, qui comprend, en tant que ledit ingrédient (A), ledit ester d'acide gras de monoglycérol (a) et ledit ester d'acide gras de polyglycérol (b) dans une plage de 20:80 à 80:20 (rapport en poids).

7. Préparation démaquillante selon l'une quelconque des revendications 1 à 6, dans laquelle ledit tensioactif non ionique liquide (d) dans ledit ingrédient (A) est choisi parmi un ester d'acide gras de polyoxyéthylène, un éther de polyoxyéthylène alkyle, un éther d'acide gras de polyoxyéthylène alkyle, un ester d'acide gras de polyoxyéthylène sorbitane, un ester d'acide gras de polyoxyéthylène sorbitol, un ester d'acide gras de polyoxyéthylène glycérol, une huile de ricin hydrogénée de polyoxyéthylène ou un éther de polyoxyéthylène-polyoxypropylène alkyle.

8. Préparation démaquillante selon l'une quelconque des revendications 1 à 7, dans laquelle une température à laquelle ladite préparation prend une forme transparente ou semi-transparente lorsque jusqu'à 100 parts en poids d'eau sont ajoutées et mélangées à 100 parts en poids de ladite préparation se trouve dans une plage de 28 à 32°C.

9. Préparation démaquillante comprenant :

(A) un tensioactif non ionique liquide ayant une chaîne de polyalkylène glycol et une valeur d'IOB de 0,75 à 1,05,
(B) 5 à 30 % en poids d'un tensioactif non ionique présentant une valeur d'IOB d'au moins 1,1,
(C) 50 à 85 % en poids d'un ingrédient huileux liquide,
(D) 12 % en poids d'eau maximum, et
(E) 0,5 à 15 % en poids d'un tensioactif non ionique, d'un alcool supérieur, d'un acide gras supérieur ou d'un dérivé de glycérol, chacun ayant une valeur d'IOB inférieure ou égale à 0,6,

dans laquelle une température à laquelle ladite préparation prend une forme transparente ou semi-transparente, lorsque que jusqu'à 100 parts en poids d'eau sont ajoutées et mélangées à 100 parts en poids de ladite préparation, se trouve dans une plage de 28 à 32°C, dans laquelle l'IOB du mélange des ingrédients (A), (B) et (E) va de 0,8 à 1,2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3161428 A
- JP 4005213 A
- JP 6219923 A
- JP 9087223 A

**Non-patent literature cited in the description**

- **Oda, Teramura et al.** Incidentally, an inorganic value and an organic value can be determined based on Organic Conceptional Diagram. *Prediction of Organic Compounds and Organic Conceptional Diagram,* 1957, vol. 11 (10), 719-725